Europäisches Patentamt

(19) European Patent Office

Office européen des brevets

(11) Numéro de publication: **0 014 106**
**B1**

(12) **FASCICULE DE BREVET EUROPEEN**

(45) Date de publication du fascicule du brevet: **08.06.83**

(51) Int. Cl.³: **C 07 C 97/24, C 07 C 79/37**

(21) Numéro de dépôt: **80400003.2**

(22) Date de dépôt: **03.01.80**

(54) **Amino-5 tétrahydro-1,2,3,4 anthraquinone et sa préparation.**

(30) Priorité: **19.01.79 FR 7901326**

(43) Date de publication de la demande:
**06.08.80 Bulletin 80/16**

(45) Mention de la délivrance du brevet:
**08.06.83 Bulletin 83/23**

(84) Etats contractants désignés:
**BE CH DE FR GB IT NL**

(56) Documents cités:
**FR - A - 658 972**
**FR - A - 2 265 724**

(73) Titulaire: **P C U K PRODUITS CHIMIQUES UGINE KUHLMANN**
**Service Propriété Industrielle Tour Manhattan**
**F-92087 Paris La Défense 2 Cédex 21 (FR)**

(72) Inventeur: **Delavarenne, Serge Yvon**
**2, rue des Alouettes**
**F-69340 Francheville le Haut (FR)**
Inventeur: **Dubreux, Bernard**
**29, avenue du Chater Bâtiment A 3**
**F-69340 Francheville le Bas (FR)**
Inventeur: **Tellier, Pierre**
**106, avenue de la Libération**
**F-69110 Sainte Foy les Lyon (FR)**

(74) Mandataire: **Houssin, Jean**
**P C U K Produits Chimiques Ugine Kuhlmann**
**Service Propriété Industrielle Tour Manhattan - Cedex 21**
**F-92087 Paris La Défense 2 (FR)**

Courier Press, Leamington Spa, England.

# 0 014 106

## Amino-5 tétrahydro-1,2,3,4 anthraquinone et sa préparation

La présente invention concerne à titre de produit industriel nouveau l'amino-5 tétrahydro-1,2,3,4 anthraquinone. Elle concerne également un procédé de préparation de ce composé par réduction de la nitro-5 tétrahydro-1,2,3,4 anthraquinone.

Les dérivés aminés des tétrahydroanthraquinones sont utiles pour la préparation d'intermédiaires dans la fabrication des colorants. De la demande de brevet français FR—A—2265724 (Mitsui Taotsu), il est connu de préparer, en partant du nitro-5 tetrahydro-1,4,4a,9a anthraquinone, l'amino-1 anthraquinone et l'amino-5 tétrahydro-1,4,4a,9a correspondant. Toutefois jusqu'a présent on ne savait pas préparer l'amino-5 tétrahydro-1,2,3,4 anthraquinone.

La demanderesse a maintenant trouvé, et c'est là l'objet de la présente invention, qu'il est possible d'obtenir l'amino-5 tétrahydro-1,2,3,4 anthraquinone par réduction de la nitro-5 tétrahydro-1,2,3,4 anthraquinone. Cette dernière est facilement préparée par nitration de la tétrahydro-1,2,3,4 anthraquinone, du tétrahydro-1,2,3,4 anthracène-diol-9,10 ou de l'hexahydro-1,2,3,4,4a,9a anthracène-dione-9,10.

La réduction selon l'invention est effectuée soit au moyen d'un composé chimique réducteur, soit par l'hydrogène en présence d'un catalyseur.

Quand la réduction est effectuée au moyen d'un composé chimique réducteur ce dernier peut être avantageusement choisi parmi les dérivés hydrogénés de l'anthraquinone tels que la tétrahydro-1,4,4a,9a anthraquinone, l'hexahydro-1,2,3,4,4a,9a anthracène-dione-9,10, le tétrahydro-1,2,3,4 anthracènediol-9,10.

Quand la réduction est effectuée par l'hydrogène on utilise comme catalyseurs ceux habituellement employés en hydrogénation. On peut citer, par exemple, ceux à base de métaux précieux comme le palladium ou le platine, ceux à base de nickel comme le nickel de Raney.

La réduction est effectuée en phase liquide, la nitro-5 tétrahydro-1,2,3,4 anthraquinone étant en solution dans un des solvants couramment utilisés pour les hydrogénations. A titre d'exemples de tels solvants, on peut citer les hydrocarbures aliphatiques, cycloaliphatiques ou aromatiques, les éthers, le tétrahydrofuranne, le dioxanne, les alcools. D'une manière générale, tous les solvants ne comportant pas un groupement fonctionnel hydrogénable dans les conditions de la réaction peuvent être utilisés.

La réduction peut être effectuée dans une large gamme de température allant de 20 à 200°C. On préfére opérer entre 60 et 130°C. Dans le cas d'une hydrogénation catalytique celle-ci est effectuée à la pression atmosphérique ou sous pression. L'hydrogénation est poursuivie jusqu'à ce que la quantité théorique d'hydrogène ait été absorbée ou jusqu'à ce qu'il n'y ait plus d'absorption d'hydrogène.

Les exemples qui suivent illustrent sans la limiter la présente invention.

Exemple 1

Dans un autoclave de 300 ml on introduit 200 ml de toluène, 2 g de nitro-5 tétrahydro-1,2,3,4 anthraquinone, 0,02 g de catalyseur à base de palladium déposé sur charbon contenant 5 % de palladium. On chauffe à 100°C et introduit de l'hydrogène sous une pression de 10 bars. On observe une absorption d'hydrogène qui cesse après 2h30 de chauffage à 100°C. On sépare le catalyseur par filtration; par concentration à sec du filtrat on obtient 1,65 g d'un solide rouge foncé constitué par de l'amino-5 tétrahydro-1,2,3,4 anthraquinone pure:

| | |
|---|---|
| Point de fusion | : 190—192°C |
| Masse | : 227 |
| Spectre IR | : $\nu CO$ à 1600 cm$^{-1}$ |
| | $\nu NH_2$ à 3350 et 3420 cm$^{-1}$ |

Spectre RMN (DMSO)=3 H aromatiques à $\varepsilon$: 6,9 à 7,6 ppm
4 H méthylène en position 1 et 4 $\delta$: 2,35 ppm
4 H méthylène en position 2 et 3 $\delta$: 1,56 ppm
2 H labiles à $\delta$: 7,7 ppm.

Exemple 2

On opère comme dans l'exemple 1 en remplaçant le toluène par de l'éthanol. L'hydrogénation dure 4 heures. On obtient 1,6 g d'amino-5 tétrahydro-1,2,3,4 anthraquinone. Point de fusion: 189—191°C.

Exemple 3

Dans l'autoclave de l'exemple 1, on introduit 100 ml d'éthanol, 25,7 g de nitro-5 tétrahydro-1,2,3,4 anthraquinone, 0,2 g de catalyseur à base de palladium déposé sur charbon contenant 5 % de palladium. On chauffe à 60°C et introduit de l'hydrogène à deux reprises sous une pression de 30 bars. L'absorption d'hydrogène cesse après 40 minutes de réaction à 60°C. Après séparation du catalyseur par filtration et concentration du filtrat, on recueille 21,7 g d'amino-5 tétrahydro-1,2,3,4 anthraquinone.

Exemple 4

Dans un réacteur en verre muni d'un agitateur, on introduit 45 ml d'alcool amylique, 3 g de nitro-5 tétrahydro-1,2,3,4 anthraquinone, 7,5 g de tétrahydro-1,2,3,4 anthracène-diol-9,10. On chauffe sous un courant d'azote pendant 90 minutes à 60°C. Après refroidissement on recueille par filtration 7,7 g d'un précipité dont l'analyse montre qu'il s'agit d'un mélange d'amino-5 tétrahydro-1,2,3,4 anthraquinone et de tétrahydro-1,2,3,4 anthraquinone. Le filtrat qui, d'après un examen par chromatographie en couche mince, contient également un mélange de ces deux composés est recyclable.

Exemple 5

On opère comme dans l'exemple 4 en remplaçant l'alcool amylique par le filtrat final de l'exemple 4. Après refroidisement, on recueille par filtration 9,8 g d'un mélange de tétrahydro-1,2,3,4 anthraquinone et d'amino-5 tétrahydro-1,2,3,4 anthraquinone.

**Revendications**

1. Amino-5 tétrahydro-1,2,3,4 anthraquinone.

2. Procédé pour la préparation de l'amino-5 tétrahydro-1,2,3,4 anthraquinone selon la revendication 1, caractérisé en ce que l'on soumet la nitro-5 tétrahydro-1,2,3,4 anthraquinone à une réduction en phase liquide au moyen d'un composé chimique réducteur ou au moyen d'hydrogène en présence d'un catalyseur.

3. Procédé selon la revendication 2 dans lequel la phase liquide est constituée par un solvant dépourvu de groups fonctionnel hydrogénable dans les conditions de la réaction.

4. Procédé selon chacune des revendications 2 et 3 dans lequel le composé chimique réducteur est le tétrahydro-1,2,3,4 anthracène-diol-9,10.

5. Procédé selon chacune des revendications 2 et 3 dans lequel le composé chimique réducteur est la tétrahydro-1,4,4a,9a anthraquinone.

6. Procédé selon chacune des revendications 2 et 3 dans lequel le catalyseur d'hydrogénation est un catalyseur à base de nickel ou de métal précieux.

7. Procédé selon les revendications 2 à 6 dans lequel la réaction est effectuée à une température comprise entre 20 et 200°C, de préférence entre 60 et 130°C.

**Patentansprüche**

1. 5-Amino-1,2,3,4-tetrahydroanthrachinon.

2. Verfahren zur Herstellung von 5-Amino-1,2,3,4-tetrahydroanthrachinon gemäß Anspruch 1, dadurch gekennzeichnet, daß man 5-Nitro-1,2,3,4-tetrahydroanthrachinon in flüssiger Phase mit Hilfe einer reduzierenden chemischen Verbindung oder mit Hilfe von Wasserstoff in Gegenwart eines Katalysators reduziert.

3. Verfahren nach Anspruch 2, dadurch gekennzeichnet, daß die flüssige Phase aus einem Lösungsmittel gebildet ist, welches frei ist von unter den Reaktionsbedingungen hydrierbaren funktionellen Gruppen.

4. Verfahren nach einer den Ansprüchen 2 und 3, dadurch gekennzeichnet, daß man als reduzierende chemische Verbindung 1,2,3,4-Tetrahydroanthracen-9,10-diol verwendet.

5. Verfahren nach einer den Ansprüchen 2 und 3, dadurch gekennzeichnet, daß man als reduzierende chemische Verbindung 1,4,4a,9a-Tetrahydroanthrachinon verwendet.

6. Verfahren nach einer den Ansprüchen 2 und 3, dadurch gekennzeichnet, daß der Hydrierkatalysator ein Katalysator auf der Grundlage von Nickel oder einer Edelmetalls ist.

7. Verfahren nach einer den Ansprüchen 2 bis 6, dadurch gekennzeichnet, daß die Reaktion bei einer Temperatur zwischen 20°C und 200°C, vorzugsweise zwischen 60°C und 130°C durchgeführt wird.

**Claims**

1. 5-amino-1,2,3,4-tetrahydro anthraquinone.

2. Process for the preparation of 5-amino-1,2,3,4-tetrahydroanthraquinone according to claim 1, characterized in that 5-nitro-1,2,3,4-tetrahydro anthraquinone is subjected to reduction in the liquid phase, by means of a reducing chemical compound, or by means of hydrogen in the presence of a catalyst.

3. Process according to claim 2, in which the liquid phase is consisting of a solvent devoid of hydrogenatable functional groups under the reaction conditions.

4. Process according to each of claims 2 and 3, in which the reducing chemical compound is 1,2,3,4-tetrahydroanthracene-9,10-diol.

5. Process according to each of claims 2 and 3, in which the reducing chemical compound is 1,4,4a,9a-tetrahydroanthraquinone.

6. Process according to each of claims 2 and 3, in which the hydrogenation catalyst is a catalyst based on nickel or a precious metal.

7. Process according to any one of claims 2 to 6, in which the reaction is effected at a temperature between 20°C and 200°C, preferably between 60°C and 130°C.